Europäisches Patentamt

(19) European Patent Office

Office européen des brevets

(11) Publication number: **0 251 727**
**A1**

# (12) EUROPEAN PATENT APPLICATION

(21) Application number: **87305704.6**

(22) Date of filing: **26.06.87**

(51) Int. Cl.⁴: **C 12 P 19/04**
**//(C12P19/04,C12R1:01)**

(30) Priority: **28.06.86 US 878730**

(43) Date of publication of application:
**07.01.88 Bulletin 88/01**

(84) Designated Contracting States:
**BE DE FR GB IT NL**

(71) Applicant: **CELGENE CORPORATION**
**86 Morris Avenue**
**Summit New Jersey 07901 (US)**

(72) Inventor: **Stirling, David I.**
**4 Lois Place**
**Fanwood New Jersey (US)**

(74) Representative: **De Minvielie-Devaux, Ian Benedict Peter et al**
**CARPMAELS & RANSFORD 43, Bloomsbury Square**
**London WC1A 2RA (GB)**

The microorganism(s) has (have) been deposited with the American Type Culture Collection under number(s) ATCC 53412.

(54) **Novel strains of facultative methylotrophic bacteria.**

(57) This invention provides Methylophilus viscogenes ATCC 53412 and variants thereof as novel strains of facultative methylotrophic bacteria.

Methylophilus viscogenes strain ATCC 53412 is capable of assimilating methanol via the ribulose monophosphate pathway to form extracellular heteropolysaccharide Poly 54 under aerobic cultivation conditions.

EP 0 251 727 A1

**Description**

NOVEL STRAINS OF FACULTATIVE METHYLOTROPHIC BACTERIA

BACKGROUND OF THE INVENTION

In the last decade the production of single-cell protein(SCP) from $C_1$-compounds has been studied extensively both by academic and industrial laboratories. Enterprises such as Mitsubishi Gas Chemical Co., Hoechst, and I.C.I. have implemented pilot plant studies, but to date only I.C.I. is operating a full scale SCP plant. "Pruteen" is the registered trademark of the I.C.I. product and is made by the fermentation of the obligate methylotrophic bacterium, Methylophilus methylotrophus, which is eventually separated and dried to a powder or granules. Methylophilus methylotrophus is an obligate methylotroph which uses the ribulose monophosphate pathway(RMP) cycle of formaldehyde fixation. Methylophilus methylotrophus Strain AS-I is a gram negative, nonpigmented rod with a single polar flagellum.

In SCP production, methanol feedstock constitutes the highest percentage of the operating costs, so that any increase in the microbial growth rate yield has a direct influence on the operating costs of SCP production. For this reason I.C.I. has applied recombinant DNA technology to increase the cell yield by altering the genome of the Methylophilus methylotrophus microorganism. The genes for the more efficient glutamate dehydrogenase nitrogen assimilation system from Escherichia coli were cloned and inserted into a Methylophilus methylotrophus strain. This strain previously had its less efficient glutamate synthase nitrogen assimilation system blocked by means of DNA mutation, as described by J. Windass et al in Nature, 287, 396(1980).

Although methanol generally is viewed as a substrate for the production of single cell protein, the factors that qualify it for SCP manufacture also recommend methanol as a potential feedstock for the production of accumulated extracellular metabolites such as exopolysaccharides. A number of microbial processes for the conversion of methanol to value-added products have been described but generally these are low-volume/high-priced compounds such as amino acids. Thus, J. Bolbot and C. Anthony in Proc. Soc. Gen. Microbial, 5, 43(1978) found that a pyruvate dehydrogenase lacking mutant of Pseudomonas AMI could accumulate the amino acids alanine and valine during growth on methanol. Y. Tani et al in Agric. Biol. Chem., 42, 2275(1978) have described the production of up to 5.2 grams per liter of L-serine from methanol employing an Arthrobacter globiformis strain. Up to the present time there has not been any report of methanol bioconversion to a commodity type of bulk chemical.

Accordingly, it is an object of this invention to provide a fermentation process for the bioconversion of $C_1$-compound to an accumulated quantity of extracellular metabolite.

It is another object of this invention to provide a rapid growth culture medium for bio-oxidation of a methanol substrate to an accumulated quantity of an exopolysaccharide.

It is another object of this invention to provide methylotrophic microorganism strains having the identifying characteristics of strain ATCC 53412 or a variant thereof.

It is a further object of this invention to provide a heteropolysaccharide which exhibits properties suitable for imparting pseudoplastic and thixotropic properties to aqueous solutions.

Other objects and advantages of the present invention shall become apparent from the accompanying description and examples.

DESCRIPTION OF THE INVENTION

One or more objects of the present invention are accomplished by the provision of novel strains of bacteria having identifying characteristics comprising:

(a) aerobic, gram-negative, rod-shaped, motile and polarly flagellated cells;
(b) methylotroph capable of assimilating methanol via the ribulose monophosphate pathway;
(c) capable of growth on fructose; and
(d) optimal growth rate at a cultivation medium temperature of 30°-43° C.

In another embodiment this invention provides a bacterial culture having the identifying characteristics of strain ATCC 53412, said culture being capable of aerobic bioconversion of methanol to an extracellular accumulation of heteropolysaccharide.

Subcultures of accession Number ATCC 53412 strain can be obtained upon request from the permanent microorganism collection of the American Type Culture Collection, 12301 Parklawn Drive, Rockville, Maryland 20852. The culture deposit in the permanent culture collection meets the requirements of the Budapest Treaty for the purposes of patent procedure.

The bacterial strains having the identifying characteristics of strain ATCC 53412 are not members of any of the known methylotrophic species such as Methylophilus methylotrophus. For purposes of taxonomic identification herein, the name Methylophilus viscogenes has been assigned to the new facultative methylotroph species which includes bacterial strains having the identifying characteristics of strain ATCC 53412 and variants thereof.

The term "methylotroph" as employed herein refers to a microorganism which is capable of growing non-autotrophically on carbon compounds having one or more carbon atoms but no carbon-carbon bonds. "Autotrophic" refers to growth on a carbon dioxide substrate.

The term "facultative methylotroph" as employed herein refers to a methylotroph which is capable of growth on one or more heterotrophic substrates, e.g., fructose.

The term "C₁-compounds" as employed herein refers to organic compounds which do not contain any carbon-carbon bonds, such as methanol, formaldehyde, formate, formamide, carbon monoxide, dimethyl ether, methylamine, dimethylamine, trimethylamine and trimethylamine N-oxide.

The term "exopolysaccharide" as employed herein refers to a polysaccharide which accumulates as an extracellular metabolite in a fermentation medium, as exemplified by xanthan gum and Poly 54.

The term "heteropolysaccharide" as employed herein refers to a polysaccharide which is composed of at least two different kinds of monosaccharidic units, such as mannose and galactose.

The term "ribulose monophosphate pathway" (RMP) as employed herein refers to the biochemical cycle in which three molecules of formaldehyde are condensed to produce either one molecule of pyruvate or one molecule of dihydroxyacetone phosphate.

Biochemical literature relating to elucidation of the ribulose monophosphate pathway and its variations include Biochem. J., 144, 465 (1974) by J. Strom et al: Sci. Prog., 62, 167 (1975) by C. Anthony; and Biochem. J., 148, 513(1975) by Colby et al.

The ribulose monophosphate pathway involves enzymes which include 6-phosphogluconate dehydrase/phospho-2-keto-3-deoxygluconate aldolase; fructose diphosphate aldolase; glucose-6-phosphate dehydrogenase; 3-hexulose phosphate synthase; phosphofructokinase; phosphoglucoisomerase; phospho-3-hexulose isomerase; phosphoriboisomerase; ribulose-5-phosphate 3-epimerase; transaldolase; transketolase; sedoheptulose diphosphate aldolase; and sedoheptulose-1,7-diphosphatase.

The RMP cycle effectively condenses 3 molecules of formaldehyde with 3 molecules of ribulose 5-phosphate to form 3 molecules of fructose 6-phosphate, which in turn regenerates 3 molecules of ribulose 5-phosphate via the cycle with the overall production of one molecule of dihydroxyacetone phosphate or pyruvic acid. These metabolites function as substrates for cellular biosynthesis.

The two key enzymes of the cycle are envisioned to be hexulose phosphate synthase and hexulose phosphate isomerase, which are the enzymes responsible for condensing formaldehyde and ribulose 5-phosphate and converting the product to fructose 6-phosphate. These are two recognized variants of the RMP cycle, i.e., the fructose bisphosphate, and the Entner-Doudoroff variant which produces pyruvic acid.

## Species Methylophilus viscogenes

A series of isolations were conducted on samples of soil and water which originated in the operating vicinity of a methanol manufacturing plant in Bishop, Texas. The samples were screened for the presence of methanol-utilizing microorganisms.

A portion of each sample was inoculated into 250 ml Erlenmeyer flasks containing methanol (0.5% v/v) and an appropriate nutrient medium, e.g., the mineral salts(MS) medium illustrated in Table I, plus one gram per liter of ammonium chloride. The flasks were incubated at various temperatures ranging from 30° to 55°C. After detectable turbidity indicative of growth occurred, 2 ml samples were removed and employed to inoculate similar sterile flasks of medium. A number of subsequent subcultures were taken for each original sample, then the cultures were streaked out on medium agar plates to obtain single microorganism-derived colonies from which pure cultures of methanol-assimilating microorganisms were obtained. Strain ATCC 39893 was isolated and obtained as a biologically pure culture in this manner. The isolation and characterization of novel strain ATCC 39893 is described in copending patent application S.N. 826,530, filed February 6, 1986, incorporated herein by reference.

The present invention Methylophilus viscogenes strain ATCC 53412 is a novel bacterium which was isolated as a spontaneous mutant from a culture of Methylophilus viscogenes strain ATCC 39893.

Strains of Methylophilus viscogenes exhibit a novel combination of properties that distinguish them from other methylotrophic bacteria. Methylophilus viscogenes strain ATCC 53412 is a facultative methylotroph which utilizes the ribulose monophosphate pathway of C₁ assimilation, and which has an exceptional growth rate doubling time of 1.5-2 hours at 35°-40°C. This growth rate doubling time is about twice the growth rate of strain ATCC 39893, and it exceeds the growth rate of any known methylotroph.

Strain ATCC 53412 grows on methanol, fructose and glucose. In addition, it will grow on a wider variety of heterotrophic substrates (e.g., succinate and pyruvate) when an exogenous energy supply in the form of formate or methanol is present. This is a property not previously described for any known microorganism. For purposes of identification, bacteria manifesting this phenomenon are herein termed "latent facultative methylotrophs".

Bacteria having the characteristics comprising those of strain ATCC 53412 are further identified by slimy growth of pale orange colonies on methanol agar media. Another identifying characteristic of a strain ATCC 53412 type of methylotrophic bacterium is a hexulose phosphate synthase/hexulose phosphate isomerase activity (J. P. Van Dijken et al; FEMS Microbiol. Lett., 4, 97, 1978) of at least about 400 nanomoles of NADH formed per minute per milligram of protein.

Another significant property of a strain ATCC 53412 type of facultative methylotroph is the ability to bioconvert a methanol substrate into an accumulated quantity of an exopolysaccharide which imparts pseudoplastic and thixotropic properties to aqueous solutions. A strain ATCC 53412 bacterium is particularly unique in its ability to produce a large quantity of accumulated exopolysaccharide in the cultivation medium, in the presence or absence of cell growth and under a variety of culture conditions. A strain ATCC 53412

bacterium has the inherent ability to divert a large percentage of available carbon source to biosynthesis of an exopolysaccharide.

A Methylophilus viscogenes ATCC 53412 type of strain can be cultured aerobically in a nutrient medium comprising a carbon source, nitrogen source, salts, and various growth promoters.

Suitable nitrogen sources include ammonium sulfate, ammonium chloride, ammonia, diammonium phosphate, ammonium nitrate, sodium nitrate, urea, corn steep liquor, casein, peptone, yeast extract, meat extract, and the like.

Suitable mineral salts include calcium salts, magnesium salts, potassium salts, phosphate salts, iron salts, manganese salts, zinc salts, copper salts, and the like. Bacterial growth promoters include soy bean protein hydrolysate, yeast extract, vitamins, and amino acids.

Cultivation of the microorganism in the nutrient medium typically is conducted aerobically at a temperature of 35°-40°C and a pH between about 6.7-7.1 by means of shaken or submerged cultivation.

The term "variant" as employed herein refers to a Methylophilus viscogenes strain which is phenotypically and/or genotypically different from the parent strain from which it is derived, and which exhibits the methylotrophic parent strain ability to biosynthesize an exopolysaccharide.

Exopolysaccharide Production

Employing the cultivation conditions previously described, a present invention strain of Methylophilus viscogenes produces and accumulates an exopolysaccharide in a high concentration and with a high rate of carbon source utilization.

Strain ATCC 53412 is capable of producing the exopolysaccharide in an amount up to about 10 grams per liter, based on the culture medium, when methanol is used as the growth carbon source. The yield of exopolysaccharide typically will vary in the range between about 30-60 percent, based on the quantity of methanol utilized.

Accordingly, in another embodiment this invention provides a process for producing a heteropolysaccharide which comprises aerobically cultivating an ATCC 53412 type of Methylophilus viscogenes strain in a nutrient medium containing a growth carbon source (e.g., a $C_1$-compound or fructose) to yield the heteropolysaccharide as an accumulated extracellular product.

In a further embodiment, this invention provides a process for producing a heteropolysaccharide which comprises aerobically cultivating Methylophilus viscogenes strain ATCC 53412 or a variant thereof in a nutrient medium containing methanol as a growth carbon source to yield heteropolysaccharide Poly 54 as an accumulated extracellular product from the culture medium.

After the completion of a fermentation run, the whole cells are removed from the broth by conventional means such as centrifugation, ultrafiltration or heat sterilization. The heteropolysaccharide is recovered from the supernatant by any convenient procedure such as freeze-drying or precipitation with a water-soluble organic solvent, e.g., acetone, methanol, ethanol, and the like. Precipitation of the heteropolysaccharide can also be effected by treatment of the solution with a calcium salt.

The crude heteropolysaccharide can be redissolved in water, and the aqueous solution then subjected to dialysis and lyophilization to provide a purified product.

The novel heteropolysaccharide Poly 54 product has an average molecular weight in the range between about 800,000 and 1,500,000.

Heteropolysaccharide Poly 54 exhibits a 0.5% viscosity of 2100 cps at 12 RPM, as measured with Brookfield RVT Viscometer Spindle No. 4 at 25°C. An aqueous solution of heteropolysaccharide Poly 54 is colorless, transparent and exhibits pseudoplastic and thixotropic viscosity properties. The aqueous solution viscosity properties are stable at temperatures up to about 130°C, and at pH values up to about 12.

Under low shear rate conditions, heteropolysaccharide Poly 54 exhibits an apparent viscosity which typically is about 2-30 times that of commercial xanthan gums. The comparative viscosity properties of heteropolysaccharide Poly 54 and commercial xanthan gum (e.g., Kelzan) under shear conditions are described in copending patent application S.N. 826,535, filed February 6, 1986, incorporated herein by reference.

Other physicochemical and structural features of heteropolysaccharide Poly 54 are as follows:

4

## A.  Constituent Sugars(mole %)

| | |
|---|---|
| glucose | 10 |
| galactose | 7-10 |
| mannose | 1-3 |
| glycuronic acid | 1-3 |

The glycuronic acid constituent comprises glucuronic acid and/or galacturonic acid and/or mannuronic acid.

Poly 54 contains no pyruvate or protein, and it contains about one acetyl group per four monosaccharide units, and about l-3 weight percent of nitrogen.

## B.  Elemental Analysis(%)*

| | |
|---|---|
| C | 39.2 |
| H | 6.1 |
| O | 43.1 |
| N | 2.17 |

*Average values, ash corrected.

The following examples are further illustrative of the present invention. The components and specific ingredients are presented as being typical, and various modifications can be derived in view of the foregoing disclosure within the scope of the invention.

For growth of methanol-assimilating bacteria a mineral salts medium(MS) (Table I) is employed. The medium is either supplemented with l g/L potassium nitrate, giving nitrate mineral salts medium (NMS) or l g/L ammonium chloride, giving ammonium mineral salts medium (AMS). The carbon source, methanol, preferably is present at a concentration between 0.2-0.5% (v/v).

For solid media, l7 g/L of Difco bacto-agar is added to the basic mineral salts medium (minus phosphates) prior to sterilization. Sterile phosphate solution is added aseptically to the sterile mineral salts medium on cooling.

Fermentation procedures are conducted in a New Brunswick Microferm (l4 liters) A l0 liter working volume is used, and methanol is added aseptically after sterilization of the fermentor. A stirring rate of 200 rpm is routinely used with an air delivery rate of two liters $m^{-1}$. The fermentor is equipped with pH and dissolved oxygen control.

Hexulose phosphate synthase/hexulose phosphate isomerase are assayed simultaneously, since there is no assay for hexulose 6-phosphate which is the product of hexulose phosphate synthase activity. Hexulose phosphate isomerase converts hexulose 6-phosphate to glucose 6-phosphate which can be estimated using glucose 6-phosphate dehydrogenase and following the concomitant NADP+ reduction at 340 nm. The enzyme assay solution contains (final conc.): phosphate buffer 50 mM, pH 7.2; magnesium chloride 2.5 mM; glucose 6-phosphate dehydrogenase 0.7 units; phosphoglucoisomerase 0.75 units; phosphoribose isomerase l.75 units; ribose 5-phosphate 5mM; NADP+ 0.25 mM; and formaldehyde 5 mM.

Any presence of hydroxypyruvate reductase is indicative of the serine pathway of formaldehyde assimilation in the microorganism. This is determined by an enzyme assay solution which contains (final conc.): potassium phosphate, l M, pH 6.3: lithium hydroxy pyruvate 0.0l M; and NADH 2 mM. NADH disappearance is measured at 340 nm.

The estimation of glycerol and dihydroxyacetone is accomplished with glycerol dehydrogenase/dihydroxyacetone reductase (glycerol:NAD+ 2-oxidoreductase, EC l.l.l.6). The enzyme is derived from Enterobacter aerogenes, and is available from Sigma Biochemicals.

For the estimation of glycerol, the reaction mixture (l.0 ml) contains 50 mole TRIS-HCl buffer (Sigma Biochemicals), pH 9.7; 0.2 mole NAD+; l unit enzyme; and l5 mole glycerol (or test solution/fermentation broth, 20-50 l). Assays are initiated by addition of substrate and followed by monitoring increasing absorbance at 340 nm in a Beckman Model 25 UV spectrophotometer.

For the estimation of dihydroxyacetone, the reaction mixture (1.0 ml) contains 50 mole phosphate buffer, pH 6.0; 0.5 mole NADH; I unit enzyme; I5 mole dihydroxyacetone (or test solution/fermentation broth, 20-50 ml). Assays are initiated by addition of substrate and followed by monitoring decreasing absorbance at 340 nm.

The exopolysaccharide (e.g., heteropolysaccharide Poly 54) is recovered after the completion of a Methylophilus viscogenes strain cultivation run. The broth is removed from the fermentor, and whole cells are removed by centrifugation (13,000 × g for 10 minutes). The exopolysaccharide usually is isolated by isopropanol precipitation from solution.

Rheological measurements on aqueous solutions of exopolysaccharides are made on a Wells/Brookfield cone/plate digital viscometer or a Rheomat 30 viscometer fitted with a coaxial cylinder sensor system.

Analysis of the monosaccharide content of an exopolysaccharide is accomplished by a hydrolysis-gas liquid chromatography method. The method involves hydrolyzing the exopolysaccharide in aqueous trifluoroacetic acid solution, then reducing with sodium borohydride and acetylating with acetic anhydride. The polyacetate derivatives of the monosaccharides are then analyzed by gas chromatography in comparison to standards.

## EXAMPLE I

This Example describes the isolation of a variant strain from a culture of Methylophilus viscogenes strain ATCC 39893.

During routine subculturing of a rifampicin-resistant strain of ATCC 39893, a spontaneous variant is formed on the growth medium. The variant is detected due to its unusual colony morphology on methanol/ammonium mineral salts agar plates.

Colonies of ATCC 39893 grown on such plates are small, round, pale orange and non-slimy in nature. Colonies of the variant strain are larger (about 4 mm in diameter), off-white to clear in color, and very slimy in nature.

The new strain (ATCC 53412) is determined to be a variant of ATCC 39893 by exhibiting rifampicin-resistance, equivalent cultural and biochemical characteristics, and finally by chromosomal DNA:DNA hybridization with ATCC 39893.

Variant strain ATCC 53412, which appears to be the product of a spontaneous mutation, is capable of constitutively synthesizing an exopolysaccharide equivalent to heteropolysaccharide Poly 54. Strain ATCC 53412 has an optimal growth rate which is 2-3 times that of ATCC 39893.

## EXAMPLE II

This Example illustrates the culturing of a Methylophilus viscogenes strain to form an accumulated quantity of exopolysaccharide metabolite.

A 500 ml inoculum of strain ATCC 53412 is grown employing MS medium, one gram per liter of ammonium chloride, and 0.5% (v/v) methanol as a growth carbon source. The flask is incubated at 37°C for two days. A 14 liter New Brunswick Microferm fermentor containing 10 liters of MS medium, one gram per liter of ammonium chloride, and 0.5% (v/v) methanol is inoculated with the above culture.

Initial fermentation conditions are as follows: 37°C, agitation 200 rpm, air flow 2 liters/minute, and pH 7.0. The pH is controlled at about 7.0 during the fermentation. Methanol is added intermittently to provide a concentration of 0.5% (v/v) whenever the fermentation medium becomes carbon exhausted. The concentration of methanol is determined by gas-liquid chromatography. After 24-36 hours of fermentation, the agitation rate is increased to 400 rpm.

The fermentation is terminated when methanol is no longer being utilized by the culture (usually 5-7 days). The highly viscous fermentation broth is then diluted with isopropanol (2:1 alcohol:broth) to facilitate precipitation of the exopolysaccharide. The resulting precipitate is filtered and then washed with 100% isopropanol, and then with 70% isopropanol. The precipitated exopolysaccharide is then dried at 55°C in a forced air oven. The dried exopolysaccharide subsequently is ground to a powder in a Wiley Mill. The physicochemical properties of the exopolysaccharide product correspond to those described hereinabove for heteropolysaccharide Poly 54.

## TABLE I

### MS MEDIUM

| | |
|---|---|
| $MgSO_4 \cdot 7H_2O$ | 1 g |
| $CaCl_2$ | 0.2 g |
| $Na_2HPO_4$ | 0.33 g |
| $KH_2PO_4$ | 0.26 g |
| FeEDTA | 5.0 mg |
| $Na_2MoO_4 \cdot 2H_2O$ | 2.0 mg |
| $CuCl_2 \cdot 2H_2O$ | 1.0 mg |
| $FeSO_4 \cdot 7H_2O$ | 500 µg |
| $ZnSO_4 \cdot 7H_2O$ | 400 µg |
| $MnCl_2 \cdot 4H_2O$ | 20 µg |
| $H_3BO_4$ | 15 µg |
| $CoCl_2 \cdot 6H_2O$ | 50 µg |
| $NiCl_2 \cdot 6H_2O$ | 10 µg |
| EDTA | 250 µg |
| $H_2O$ - | 1 liter  pH 6.8 |

**Claims**

1. A biologically pure culture having the identifying characteristics of Methylophilus viscogenes strain ATCC 53412.

2. A bacterial culture exhibiting taxonomic characteristics of Methylophilus viscogenes strain ATCC 53412, said culture being capable of aerobic fermentation of methanol to an extracellular heteropolysaccharide.

3. A bacterial culture in accordance with claim 2 wherein the extracellular heteropolysaccharide is heteropolysaccharide Poly 54.

4. Methylophilus viscogenes strain ATCC 53412.

5. A process for producing an exopolysaccharide which comprises cultivating Methylophilus viscogenes strain ATCC 53412 or a variant thereof under aerobic conditions in a nutrient medium containing a growth carbon source.

6. A process in accordance with claim 5 wherein the growth carbon source is a $C_1$-compound.

7. A fermentation process which comprises aerobically cultivating Methylophilus viscogenes strain ATCC 53412 or a variant thereof in a nutrient medium containing methanol as a growth carbon source to produce an accumulated quantity of extracellular heteropolysaccharide product.

8. A fermentation process which comprises aerobically cultivating Methylophilus viscogenes strain

ATCC 53412 in a nutrient medium containing methanol as a growth carbon source to produce an accumulated quantity of extracellular heteropolysaccharide Poly 54.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | RESEARCH DISCLOSURE, Vol. 264, April 1986, pages 182-184, Abstract no. 26420, Kenneth Mason Publications Ltd, Hampshire, GB; "Heteropolysaccharides" * Whole document * | 1-8 | C 12 P 19/04 // (C 12 P 19/04 C 12 R 1:01 ) |
| P,X | EP-A-0 215 623 (CELANESE CORP.) * Page 2, lines 51-54 * | 1-8 | |

---

**TECHNICAL FIELDS SEARCHED (Int. Cl.4)**

C 12 P
C 12 N

The present search report has been drawn up for all claims

| Place of search | Date of completion of the search | Examiner |
|---|---|---|
| THE HAGUE | 12-10-1987 | SOMERVILLE F.M. |